# EUROPEAN PATENT APPLICATION

(11) **EP 1 297 797 A2**
(43) Date of publication of application: **02.04.2003**
(21) Application number: 02256689.7
(22) Date of filing: 25.09.2002
(51) Int. Cl.: A61F 2/01

(54) **Vascular filter system with encapsulated filter**

(30) Priority: 27.09.2001 US 965401
(71) Applicant: Cordis Corporation, Miami Lakes Florida 33014 (US)
(72) Inventor: Boucher, Don, Boynton Beach, FL 33437 (US)
(74) Representative: Belcher, Simon James

(57) **Abstract**

A vascular filter system with encapsulated filter comprising a guidewire, a tip attached near the distal end of the guidewire, a filter attached near the proximal end of the tip, and actuating means for causing the filter to move between a smaller first diameter for insertion into the lumen of a vessel, and a second larger diameter for expanding to substantially equal the diameter of the lumen and to be placed in generally sealing relationship with the lumen. The actuating means comprises a catheter and a capsule attached near the distal end of the catheter. The capsule/tip combination protects the filter during insertion, and facilitates pushing and tracking the system through the vasculature. The design of the capsule and catheter increases column strength and reduces profile, thereby enhancing flexibility of the system in tortuous anatomy, and minimizing deployment forces.

## Description

The present invention relates to the treatment of vascular disease, and more particularly to a vascular filter system for use during medical procedures.

Percutaneous transluminal coronary angioplasty (PTCA), stenting and atherectomy are therapeutic medical procedures used to increase blood flow through the coronary arteries. These procedures can often be performed as alternatives to coronary bypass surgery. Percutaneous transluminal angioplasty (PTA) and stenting can often be performed as alternatives to carotid endarterectomy, and femoral-popliteal bypass procedures. In PTCA or PTA procedures, the angioplasty balloon is inflated within the stenosed vessel, at the location of an occlusion, in order to shear and disrupt the wall components of the vessel to obtain an enlarged lumen. In stenting, an endoluminal prosthesis is implanted in the vessel to maintain patency following the procedure. In atherectomy, a rotating blade is used to shear plaque from the arterial wall.

One of the complications associated with all these techniques is the accidental dislodgment of plaque, thrombus or other embolic particulates generated during manipulation of the vessel, thereby causing occlusion of the narrower vessels downstream and ischemia or infarct of the organ which the vessel supplies. Such emboli may be extremely dangerous to the patient, and may result in myocardial infarction, stroke or limb ischemia. In 1995, Waksman et al. disclosed that distal embolization is common after directional atherectomy in coronary arteries and saphenous vein grafts. See Waksman et al., American Heart Journal 129(3): 430-5 (1995). This study found that distal embolization occurs in 28% (31 out of 111) of the patients undergoing atherectomy. In January 1999, Jordan, Jr. et al. disclosed that treatment of carotid stenosis using percutaneous angioplasty with stenting procedure is associated with more than eight times the rate of microemboli seen using carotid endarterectomy. See Jordan, Jr. et al. Cardiovascular Surgery 7(1): 33-8 (1999). Microemboli, as detected by transcranial Doppler monitoring in this study, have been shown to be a potential cause of stroke. The embolic materials include calcium, intimal debris, atheromatous plaque, and thrombi.

In order to initiate these procedures, one must first introduce a guidewire into the lumen of the vessel to serve as a conduit for other interventional devices, such as angioplasty balloons and stent delivery systems. This guidewire must be advanced into a position past the location of the occlusion. Guidewires must be capable of traversing tortuous pathways within the body, consisting of bends, loops and branches. For this reason, guidewires need to be flexible, but they should also be sufficiently stiff to serve as conduits for other devices. In addition, they must be "torqueable" to facilitate directional changes as they are guided into position. Guidewires are well known in the art, and are typically made of stainless steel, tantalum or other suitable materials, in a variety of different designs. For example, guidewires are disclosed in US-4545390 and US-4619274 in which the distal segment is tapered for greater flexibility. The tapered section may be enclosed in a wire coil, typically a platinum coil, which provides increased column strength and torqueability. Another design is disclosed in US-5095915, where the distal segment is encased in a polymer sleeve with axially spaced grooves to provide bending flexibility.

Vascular filters are also well known in the art, especially vena cava filters, as disclosed in US-4727873 and US-4688553. There is also a substantial amount of medical literature describing various designs of vascular filters and reporting the results of clinical and experimental use thereof. See, for example, the article by Eichelter and Schenk, entitled "Prophylaxis of Pulmonary Embolism," Archives of Surgery, Vol. 97 (August, 1968). See, also, the article by Greenfield, et al, entitled "A New Intracaval Filter Permitting Continued Flow and Resolution of Emboli", Surgery, Vol. 73, No. 4 (1973).

Vascular filters are often used during a postoperative period, when there is a perceived risk of a patient encountering pulmonary embolism resulting from clots generated peri-operatively. Pulmonary embolism is a serious and potentially fatal condition that occurs when these clots travel to the lungs. The filter is therefore typically placed in the vena cava to catch and trap clots before they can reach the lungs.

Many of the vascular filters in the prior art are intended to be permanently placed in the venous system of the patient, so that even after the need for the filter has passed, the filter remains in place for the life of the patient. A stainless steel filtering device is disclosed in US-3952747 which is permanently implanted transvenously within the inferior vena cava. This device is intended to treat recurrent pulmonary embolism. Permanent implantation is often deemed medically undesirable, but it is done because filters are implanted in patients in response to potentially life-threatening situations.

To avoid permanent implantation, it is highly desirable to provide an apparatus and method for preventing embolization associated with angioplasty, stenting or other procedures. In particular, it is desirable to provide a device which can be temporarily placed within the vascular system to collect and retrieve plaque, thrombus and other embolic particulates which have been dislodged during angioplasty, stenting or other procedures. Such a device is removed at the end of the procedure. Guidewire-based filters are disclosed in US-6179861 and US-6001118 in which the filter resembles a windsock and is supported by one or more articulated support hoops. Guidewire-based filter devices are disclosed in US-5814064 and US-5827324 in which the filter is expanded to a predetermined diameter through the introduction of a fluid or a gas. Guidewire-based filters are disclosed in US-6168604 and US-6152946 in which the diameter of the filter is controlled by advancing and retracting a sheath over the filter component.

One concern commonly encountered with these devices is that their profile or diameter makes it is difficult to push and track these devices through the vasculature to reach the treatment site. A related concern commonly encountered with these devices is that the leading or training edges of the system tend to get hung up on the anatomy as they track through the vasculature to reach the treatment site. Another concern commonly encountered with these devices is that they are not sufficiently flexible to be delivered through tortuous anatomy. Finally, another concern commonly encountered with these devices is that the force to deploy the filter can be high and can cause procedural difficulty, especially when the chronic outward force exerted by the filter causes the filter to become embedded in the delivery sheath.

The prior art has yet to disclose any guidewire-based vascular filters which can be used to address the clinical problems of poor tracking through the vasculature, insufficient flexibility for delivery through tortuous anatomy, and high filter deployment forces.

The present invention provides for a vascular filter system with an encapsulated filter, which can be used to address the clinical problems of poor tracking through the vasculature, insufficient flexibility for delivery through tortuous anatomy, and high filter deployment forces, as briefly described above.

In accordance with one aspect, the present invention is directed to a vascular filter system with an encapsulated filter for insertion into a lumen of a vessel, comprising a guidewire, a tip attached near the distal end of the guidewire, a vascular filter attached near the proximal end of the tip, a porous flexible filter membrane attached to the vascular filter, and actuating means for causing the vascular filter to move between a smaller first diameter for insertion into the lumen and a larger second diameter for expanding to substantially equal the diameter of the lumen and to be placed in generally sealing relationship with the lumen. The actuating means comprises a catheter, and a capsule attached near the distal end of the catheter. The tip has a minimum outer diameter, which is as close as possible to the outer diameter of the guidewire. The tip has a maximum outer diameter, which is as close as possible to the outer diameter of the capsule. The catheter has an outer diameter, which is as close as possible to the outer diameter of the guidewire.

The proximal end of the guidewire is inserted into the distal end of the capsule/catheter assembly and advanced until the proximal end of the tip and the distal end of the capsule are substantially in contact. At this point, the filter is collapsed within the capsule. Then, the vascular filter system with encapsulated filter may be inserted into the lumen of a vessel. The catheter/capsule assembly is then retracted back over the guidewire, and the filter is deployed. The vascular filter with a porous flexible filter membrane is then used to capture embolic particulates released during the balance of the interventional procedure. When the procedure is complete, the catheter/capsule assembly is again advanced over the guidewire until the proximal end of the tip and the distal end of the capsule are substantially in contact. At this point, the collapsed filter is again within the capsule. The vascular filter system with encapsulated filter may then be withdrawn from the lumen.

An advantage of the present invention is that the tip and capsule create a smooth transition from the small diameter of the guidewire to the larger diameter of the capsule covering the collapsed basket, thereby avoiding the problem of leading and trailing edges of the system getting hung up on the anatomy as they track through the vasculature. Also, the braided design of the capsule and catheter increases column strength and reduces profile, thereby enhancing pushability and trackability of the system. Finally, the braided capsule avoids embedded filters which may result in high deployment forces.

The vascular filter system with encapsulated filter of the present invention is designed to address the clinical problems of poor tracking through the vasculature, insufficient flexibility for delivery through tortuous anatomy, and high filter deployment forces.

The vascular filter system with encapsulated filter comprises a guidewire, a tip attached near the distal end of the guidewire, a vascular filter attached near the proximal end of the tip, a porous flexible filter membrane attached to the vascular filter, and actuating means for causing the vascular filter to move between a smaller first diameter for insertion into the lumen and a larger second diameter for expanding to substantially equal the diameter of the lumen and to be placed in generally sealing relationship with the lumen. The actuating means comprises a catheter, and a capsule attached near the distal end of the catheter. The tip has a minimum outer diameter, which is as close as possible to the outer diameter of the guidewire. The tip has a maximum outer diameter, which is as close as possible to the outer diameter of the capsule. The catheter has an outer diameter, which is as close as possible to the outer diameter of the guidewire.

The proximal end of the guidewire is inserted into the distal end of the capsule/catheter assembly and advanced until the proximal end of the tip and the distal end of the capsule are substantially in contact, and the filter is collapsed within the capsule. The vascular filter system with encapsulated filter may then be inserted into the lumen of a vessel. The tip and capsule create a smooth transition from the small diameter of the guidewire to the larger diameter of the capsule, thereby avoiding the problem of leading and trailing edges of the system getting hung up on the anatomy as they track through the vasculature. Also, the braided design of the capsule and catheter increases column strength and reduces profile, thereby enhancing pushability and trackability of the system. Finally, the braided capsule avoids embedded filters which may result in high deployment forces.

The catheter/capsule assembly is then retracted back over the guidewire, and the filter is deployed and used to capture embolic particulates released during the balance of the interventional procedure. When the procedure is complete, the catheter/capsule assembly is again advanced over the guidewire until the proximal end of the tip and the distal end of the capsule are substantially in contact. Then, the collapsed filter is again within the capsule. The vascular filter system with encapsulated filter may then be withdrawn from the lumen.

Embodiments of the invention will now be described by way of example with reference to the accompanying drawings, in which:

Figure 1 is a simplified, cross-sectional view of an exemplary embodiment of the vascular filter system with encapsulated filter, with the filter in the collapsed position within the capsule, in accordance with the present invention.

Figure 2 is a simplified, cross-sectional view of an exemplary embodiment of the vascular filter system with encapsulated filter, with the filter in the deployed or expanded position within the lumen, in accordance with the present invention.

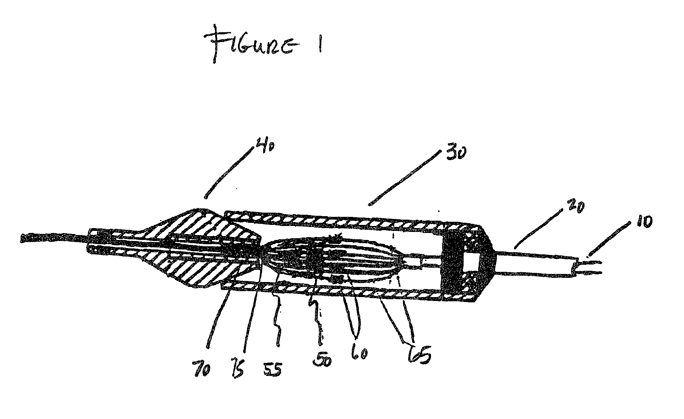

Referring to the figures wherein like numerals indicate the same element throughout the views, there is shown in Figure 1, a vascular filter system with encapsulated filter system made in accordance with the present invention. The vascular filter system with encapsulated filter comprises a guidewire 10, a tip 40 attached near the distal end of the guidewire, a filter 50, attached near the proximal end of the tip 40, and a porous flexible filter membrane 55 attached to the filter 50. The filter further comprises a plurality of markers 60, attached near the midpoint of the filter struts 65, and a filter distal marker band 70 attached near the distal end of the filter. The vascular filter system with encapsulated filter further comprises a catheter 20, and a capsule 30 attached near the distal end of the catheter 20.

Figure 1 shows an exemplary embodiment of the vascular filter system with encapsulated filter made in accordance with the present invention. As illustrated in Figure 1, the distal end of the capsule 30 has been inserted over the guidewire 10, and the filter 50, until the distal end of the capsule 30 is substantially in contact with the proximal portion of the tip 40. The filter 50 is then collapsed inside the capsule 30, and has achieved a smaller first diameter. The capsule 30 is attached near the distal end of the catheter 20.

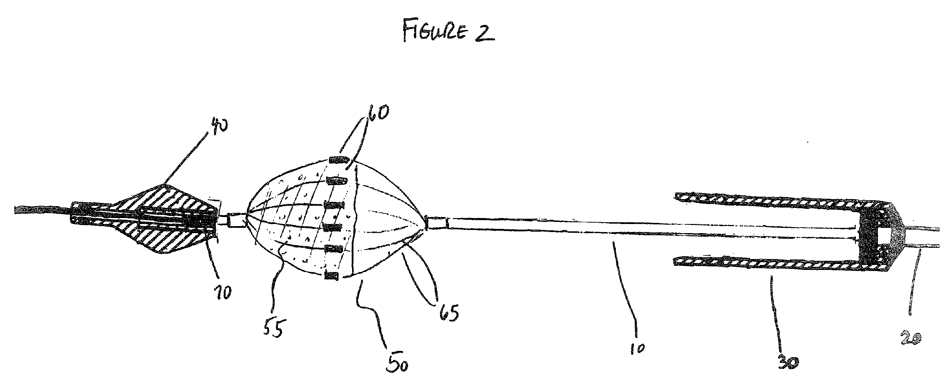

Figure 2 shows an exemplary embodiment of the vascular filter system with encapsulated filter made in accordance with the present invention. As illustrated in Figure 2, the capsule 30 and catheter 20 have been retracted back over the guidewire 10, and the filter 50 has achieved a larger second diameter. The filter struts 65 have opened, and the filter marker bands 60 may be used to identify the location of the filter within the vessel. The filter distal marker band 70 may be used to identify the endpoint of the filter. The porous flexible filter membrane 55 may now capture embolic particulates which may be released during the interventional procedure.

As illustrated in Figures 1 and 2, the vascular filter system with encapsulated filter may be used to smoothly introduce a vascular filter system through tortuous anatomy and into position beyond the location of a lesion or other obstruction. The proximal end of the guidewire 10 is inserted into the distal end of the capsule 30, and advanced through the capsule 30 and attached catheter 20 until the distal end of the capsule 30 is substantially in contact with the proximal portion of the tip 40. At this point, the filter 50 is collapsed within the capsule 30, and the filter 50 has achieved a smaller first diameter, as illustrated in Figure 1. Then the system may be introduced into the lumen of a vessel. The guidewire 10 to tip 40 to capsule 30 transition is sufficiently smooth to avoid the problem of leading and trailing edges of the system getting hung up on the anatomy as they track through the vasculature. Also, the braided design of the capsule 30 and catheter 20 increases column strength and reduces profile, thereby enhancing pushability and trackability of the system. Finally, the braided capsule 30 avoids embedded filters which can result in high deployment forces. As illustrated in Figure 2, once the capsule 30 is positioned beyond the location of the lesion or obstruction, as may be verified by the position of the filter distal marker band 70, the capsule 30 and catheter 20 may be retracted back over the guidewire 10, until the filter 50 is deployed and has achieved a second larger diameter, as may be verified by the position of the filter marker bands 60. Additional interventional devices such as angioplasty balloons and stents may be introduced over the guidewire 10 to therapeutically treat the lesion or obstruction. The filter 50 and the porous flexible filter membrane 55 are now in position to capture embolic particulates which may be generated during the interventional procedure. When the procedure is complete, the capsule 30 and catheter 20 may be advanced over the guidewire 10 until the capsule 30 and tip 40 are substantially in contact. At this point, the filter 50 is collapsed within the capsule 30, and the filter 50 and porous flexible filter membrane 55 have captured and contained the embolic particulates generated during the procedure. The system may then be withdrawn from the lumen of the vessel.

The filter 50 and the guidewire 10 may be made from any number of suitable materials, and are preferably made from a superelastic alloy such as Nickel-Titanium. The porous flexible filter membrane 55 on the filter 50 may be made from any number of suitable materials, and is preferably made from a flexible polymeric material with elastomeric properties chosen from a group consisting of polyurethane, polyethylene or a co-polymer thereof. The porous flexible filter membrane 55 on the filter 50 may comprise any number and configuration of pores and preferably comprises regularly-spacer laser-formed holes wherein the pore size is from about 20 to about 300 microns. The filter marker bands 65 and the filter distal marker band 70 may be made from any suitable material, and are preferably made from radiopaque materials such as tantalum. The tip 40 may be made from any suitable material, and is preferably made from a moulded material such as a block copolymer sold under the trade mark Pebax. The capsule 30 may be made from any suitable material, and is preferably made from a braided material, and more preferably is made from braided polyimid. The catheter 20 may be made from any suitable material, and is preferably made from a braided material, and more preferably is made from braided nylon and PTFE.

## Claims

1. A vascular filter system for insertion into a lumen of a vessel, said system comprising:
a) a guidewire having an outer diameter, a proximal end and a distal end;
b) a tip having a proximal portion and a distal portion, a maximum outer diameter and a minimum outer diameter, and an inner diameter, with said distal portion of said tip attached near said distal end of said guidewire;
c) a filter attached near said proximal portion of said tip, said filter comprising a proximal portion, a distal portion, and a plurality of struts extending therebetween, said struts having lengths, said struts further comprising proximal portions and distal portions and midpoints therebetween, said filter further comprising a porous flexible filter membrane having a length, with said porous flexible filter membrane connected to said filter distal portion and said plurality of struts, wherein said porous flexible filter membrane length is less than said lengths of said plurality of struts, said filter having a smaller first diameter for insertion into said lumen, and a second larger diameter for expanding to substantially equal the diameter of said lumen and to be placed in generally sealing relationship with said lumen; and
d) actuating means for causing said filter to move between said smaller first diameter and said larger second diameter, said actuating means comprising a catheter having an outer diameter and an inner diameter, a proximal end and a distal end, and an inner lumen; said actuating means further comprising a capsule having an outer diameter and an inner diameter, a proximal end and a distal end, and an inner lumen, with said proximal end of said capsule attached near said distal end of said catheter, and said lumen of said capsule being in fluid communication with said lumen of said catheter.

2. The vascular filter system according to claim 1, wherein at least one of said guidewire and the filter is made from nickel-titanium alloy.

3. The vascular filter system according to claim 1, wherein said tip is a moulded tip.

4. The vascular filter system according to claim 1, wherein said distal portion of said tip has said minimum diameter of said tip, which is as close as possible to said outer diameter of said guidewire, and said proximal portion of said tip has said maximum diameter of said tip, which is as close as possible to said outer diameter of said capsule.

5. The vascular filter system according to claim 1, wherein said filter further comprises at least one marker band attached near said midpoints of said plurality of struts, said filter further comprising at least one marker band attached near said distal portion of said filter.

6. The vascular filter system according to claim 1, wherein said porous flexible filter membrane is made from a flexible polymeric material chosen from a group consisting of polyurethane, polyethylene or a co-polymer thereof.

7. The vascular filter system according to claim 6, wherein the pore size of said porous flexible filter membrane is from about 20 to about 300 µm.

8. The vascular filter system according to claim 1, wherein said catheter is made from a polymeric material, preferably a braided polymer, especially a braided polyimid.

9. The vascular filter system according to claim 1, wherein said outer diameter of said catheter is as close as possible to said outer diameter of said guidewire.

10. The vascular filter system according to claim 1, wherein said capsule is made from a polymeric material.

11. The vascular filter system according to claim 10, wherein said capsule is made from a braided material, preferably from braided nylon and PTFE.

12. The vascular filter system according to claim 1, wherein said outer diameter of said capsule is as close as possible to said maximum diameter of said tip.

13. A vascular filter system which comprises a vascular filter and a guidewire to which the filter is attached, the guidewire having an outer diameter, a proximal end and a distal end, and the vascular filter having a proximal end and a distal end, with the vascular filter attached near to the distal end of said guidewire, the filter having a smaller first diameter and a second larger diameter, the system including a tip having a proximal portion and a distal portion, a maximum outer diameter and a minimum outer diameter, and an inner diameter, with said distal portion of said tip attached near said distal end of said guidewire and said proximal portion of said tip attached near said distal end of said filter; wherein said distal portion of said tip has said minimum diameter of said tip, which is as close as possible to said outer diameter of said guidewire, and said proximal portion of said tip has said maximum diameter of said tip, which is as close as possible to said smaller first diameter of said vascular filter.
